# DEMANDE DE BREVET EUROPEEN

(11) **EP 1 685 805 A1**
(43) Date de publication de la demande: **02.08.2006**
(21) Numéro de dépôt: 05447012.5
(22) Date de dépôt: 31.01.2005
(51) Int. Cl.: A61B 18/14

(54) **Dispositif d'épilation par électrolyse.**

(71) Demandeur: FACO S.A., B-4020 Wandre (BE)
(72) Inventeur: Smal, Olivier, 4053 Embourg (BE)
(74) Mandataire: Van Malderen, Michel

(57) **Abrégé**

Un dispositif d'épilation par électrolyse comportant une unité de gestion centrale (4) équipée d'un microprocesseur, et des patchs (8) d'épilation de taille variable adaptés aux différentes parties du corps à traiter ainsi que des patchs de contact (10) pour créer le courant électrolytique sur la peau, dans lequel ledit dispositif comporte un système d'identification des patchs d'épilation permettant au microprocesseur de l'unité de gestion centrale de les identifier et de réguler la durée d'application du courant en fonction de l'intensité choisie par l'utilisateur afin d'administrer une dose d'énergie spécifiquement associée à la taille de patch d'épilation choisi.

## Description

### Objet de l'invention

La présente invention se situe dans le domaine des dispositifs d'épilation dits permanents ou de longue durée, et en particulier des dispositifs d'épilation par électrolyse.

### Etat de la technique

L'épilation par électrolyse est bien connue de l'homme de métier. Cette technique consiste à véhiculer un courant continu à peine perceptible vers la base du follicule pileux, ce qui entraîne la neutralisation de la papille dermique qui nourrit le poil et ralentit fortement sa repousse. Ce type de dispositif d'épilation ne vise donc pas uniquement le poil apparent mais aussi la racine pour une épilation durable, dite « de longue durée » ou encore « définitive ».

Le poil pousse dans une poche appelée follicule. Selon les zones, la densité de follicules varie entre environ 60 follicules/cm² pour les jambes à 500 follicules/cm² pour la moustache.

Les poils poussent par cycle : durant la phase de croissance (ou phase anagène), le poil est nourri par la papille dermique avant d'entrer ensuite dans une phase de transition (ou phase catagène) et enfin dans une phase de repos (ou phase tellogène) au cours de laquelle le poil ne pousse pas car il est séparé de la papille dermique et il tombe, après, le follicule se remet en contact avec la papille dermique et un nouveau cycle recommence. Il faut environ trois semaines au poil pour pousser de la base du follicule à la surface de la peau et donc pour être visible. Autrement dit, un poil qui commence à pousser n'apparaîtra que trois semaines plus tard.

A partir du moment où le poil apparaît à la surface de la peau, la période de croissance continue encore environ treize semaines. Pour que le traitement cosmétique soit efficace, il faut que les poils soient traités durant cette phase anagène, quand les papilles dermiques sont encore actives, c'est la condition sine qua non pour stopper réellement le processus de pousse du poil.

Par ailleurs, les poils poussent en cycle décalé : sur une même zone, certains poils se trouvent dans leur phase de croissance (anagène) et d'autres dans leur phase de repos (tellogène) ; c'est la raison pour laquelle les premiers résultats ne sont obtenus qu'à partir de quelques semaines d'application du dispositif de l'invention sur les parties du corps à épiler.

Les dispositifs d'épilation définitive ou de longue durée, par électrolyse ou par une méthode apparentée sont notamment divulgués par le brevet US-60/397,406 qui décrit un générateur de courant électrique qui transmet un courant à un patch collé sur la peau. Les documents DE 3 426 141 et US 5,534,003 décrivent également des inventions selon le même principe.

Le document US 5,522,814 décrit une méthode d'épilation à haute fréquence.

Le document FR-2 680 965 décrit un appareil et un procédé pour le traitement cosmétique de la peau en vue d'une épilation de longue durée qui consiste à induire un courant électromagnétique de haute fréquence à travers la peau à l'aide d'un gel conducteur chargé de produit traitant, le courant électromagnétique amenant ce produit à pénétrer les pores de la peau.

Le document FR-2 595 239 divulgue un appareil d'épilation permanente comportant un générateur de courant haute fréquence produisant un courant haute fréquence haché en paquets d'ondes alternées pouvant être appliqué sur la peau.

Le problème retrouvé dans tous les dispositifs connus de l'art antérieur réside dans le fait que l'utilisateur peut lui-même choisir à la fois l'intensité et la durée, donc l'énergie d'application du courant d'électrolyse. L'énergie à dispenser doit être adaptée à la taille des surfaces du corps à traiter et à la sensibilité de celles-ci. Ceci entraînait bien souvent une utilisation inadéquate de ce type d'appareil pouvant entraîner des irritations de la peau.

Il fallait donc remédier à ce problème en proposant un dispositif capable d'administrer une dose d'énergie précise en fonction de la surface des patchs utilisés et il était donc devenu nécessaire d'élaborer un dispositif capable d'identifier des patchs de taille différente afin de permettre un dosage précis de l'énergie administrée à une surface donnée.

### Buts de l'invention

La présente invention vise à fournir un dispositif d'épilation par électrolyse comportant une unité de gestion centrale équipée d'un microprocesseur et un ensemble de patchs (épilation et de contact) capable d'appliquer des intensités de courant précises pendant des durées précises afin d'appliquer des doses d'énergie constantes par patch identifié. Pour arriver à cette fin, l'invention propose un système de reconnaissance des patchs permettant l'application de doses ciblées pour chaque type d'utilisation.

### Résumé de l'invention

La présente invention divulgue un dispositif d'épilation par électrolyse comportant une unité de gestion centrale équipée d'un microprocesseur, et des patchs d'épilation de taille variable adaptés aux différentes parties du corps à traiter ainsi que des patchs de contact permettant de créer un courant d'électrolyse sur la peau, caractérisé en ce que ledit dispositif comporte un système d'identification des patchs d'épilation permettant au microprocesseur de l'unité de gestion centrale de les identifier et de réguler la durée d'application du courant en fonction de l'intensité choisie par l'utilisateur afin d'administrer une dose d'énergie spécifiquement associée à la taille du patch d'épilation utilisé.

Selon des modes particuliers de réalisation, l'invention comporte l'une ou plusieurs des caractéristiques suivantes :
- le dispositif d'épilation comporte en outre un stylet de précision,
- ledit stylet de précision possède plusieurs têtes d'application interchangeables,
- l'unité centrale possède un système visuel permettant à l'utilisateur d'identifier les parties traitées,
- ledit dispositif de d'identification (5, 6, 7) comporte un circuit oscillant,
- ledit dispositif de d'identification (5, 6, 7) comporte des résistances.

L'invention divulgue par ailleurs, un procédé de traitement cosmétique à l'aide du dispositif de la revendication 1 comportant les étapes suivantes:
- génération d'un courant continu de faible intensité par une unité de gestion centrale,
- identification du patch d'épilation (8) via le système d'identification du dispositif de la revendication 1,
- adaptation de l'intensité du courant par l'utilisateur en fonction de sa propre sensibilité,
- réglage automatique de la durée de traitement par l'unité de gestion centrale (4).

Enfin, l'invention divulgue l'utilisation du dispositif selon la revendication 1 pour l'épilation durable.

### Brève description des figures

La figure 1 représente le schéma de connexion des patchs sur l'unité de gestion centrale.

La figure 2 représente le schéma de connexion du stylet de précision sur l'unité de gestion centrale.

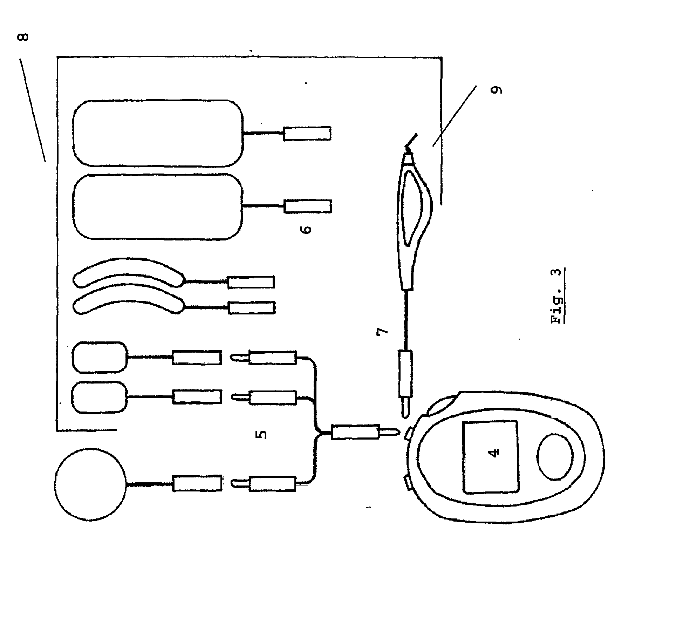

La figure 3 représente une vue de l'ensemble du dispositif avec l'unité centrale, ses patchs et le stylet de précision.

### Description détaillée de l'invention

Le dispositif d'épilation par électrolyse comporte une unité de gestion centrale 4 équipée d'un microprocesseur, différents patchs 8 de taille différente adaptés aux zones d'application (visage, jambes, zones sensibles) et des patchs de contact 10 qui permettent de fermer le circuit pour faire passer le courant d'électrolyse par la peau. L'ensemble comporte également un stylet de précision 9 avec plusieurs têtes interchangeables.

Le dispositif d'épilation par électrolyse de la présente invention est équipé d'un dispositif automatique d'identification des patchs. L'unité de gestion centrale 4 va donc « reconnaître » les patchs 8 ou le stylet 9 qui seront branchés et les zones à traiter s'afficheront sur le mannequin de l'écran de cette unité 4. L'unité de gestion centrale 4 permet de varier l'intensité du courant en fonction de la sensibilité de la peau. Ensuite, le dispositif de l'invention adaptera automatiquement la durée du traitement. L'unité centrale 4 comporte un sablier qui affiche sur l'écran la durée restante du traitement.

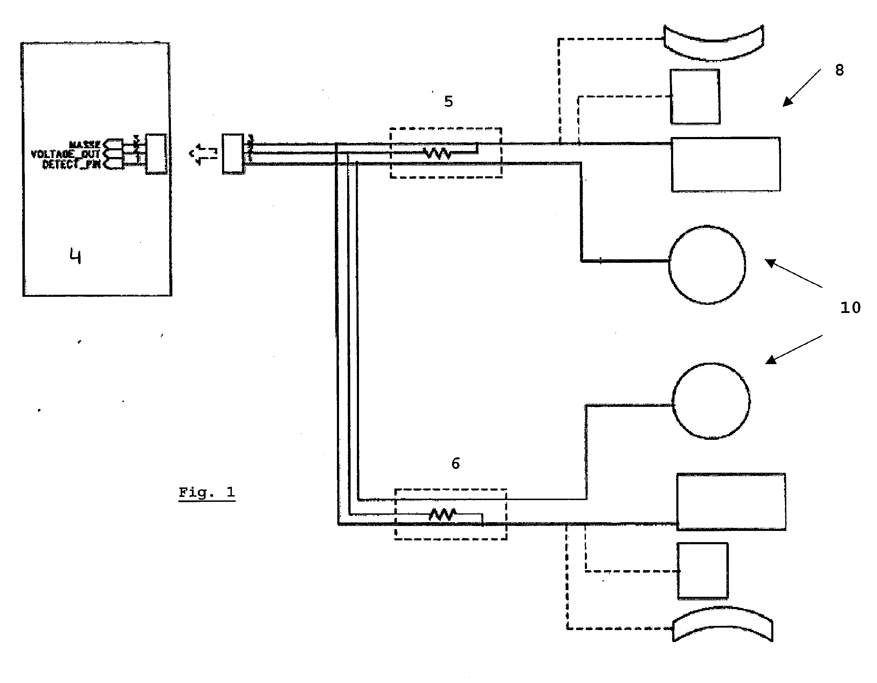

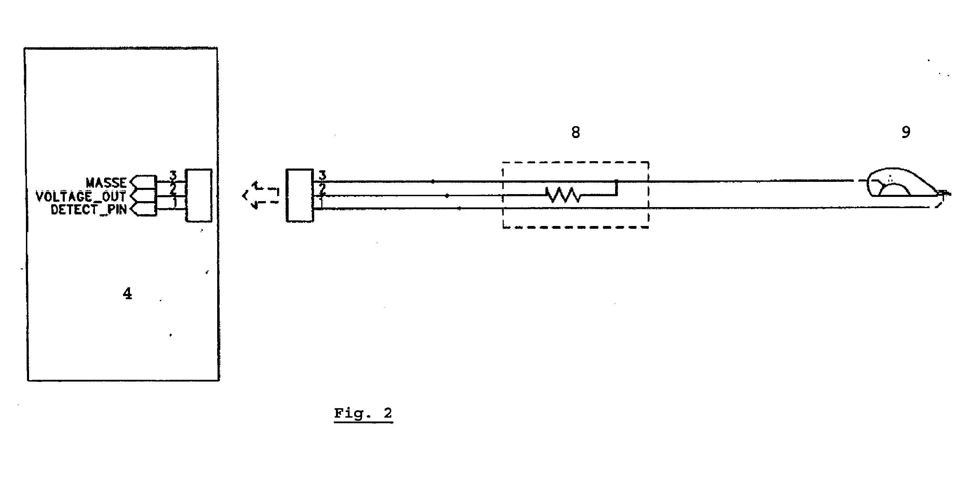

Pour effectuer l'identification des patchs 8 ou du stylet 9 utilisé, les prises de ceux-ci sont munis d'une résistance électrique (5 ,6, 7), ce qui permet à l'appareil, à l'aide d'un circuit oscillant, de détecter quel type de patch est utilisé (figures 1 et 2).

Le circuit est composé d'une résistance (5, 6, 7) située dans le patch ou le stylet à reconnaître et d'une capacité située sur l'unité de gestion centrale. Le microprocesseur de cette unité 4 reconnaît la fréquence du circuit oscillant et adapte l'énergie délivrée par l'appareil en fonction de la taille et de la fonctionnalité donnée au patch choisi 8.

Le schéma de connexion des patchs 8 à l'unité centrale 4 est représenté dans la figure 1. Pour reconnaître la fréquence du circuit oscillant et adapter l'énergie délivrée, deux fils électriques 2 et 3 sont raccordés aux patchs 8 ou au stylet 9 (voir figure 2) afin de fermer le circuit oscillant. Le fil 3 est utilisé pour fermer le circuit via la peau de l'utilisateur et le patch de contact 10. Afin de délivrer une énergie constante à l'utilisateur, le microprocesseur adapte le temps en fonction de l'intensité du courant choisie par l'utilisateur ; ainsi, quels que soient les changements d'intensité effectués par l'utilisateur durant la période de traitement, l'intégrale du courant sur l'intervalle de temps de fonctionnement reste constante et l'appareil délivre donc une dose d'énergie constante pour chaque type de patchs.

Les utilisateurs de ce type de dispositif ont généralement des sensibilités très différentes par rapport au nombre de milliampères appliqués à leur peau. C'est la raison pour laquelle chacun règle l'intensité du courant à son propre niveau de confort. L'unité de gestion 4 gère ensuite la durée.

Le tableau suivant montre quelques exemples de résistances différentes permettant de reconnaître les patchs :

| **Patch et stylet** | **Résistance** |
|---|---|
| Patch pour les jambes | 430000 Ohm |
| Patch pour parties sensibles | 150000 Ohm |
| Patch pour visage | 47000 Ohm |
| Stylet de précision | 15000 Ohm |

Dans chaque mode de fonctionnement, l'intensité de courant multipliée par le temps est une constante

| **Mode** | **Courant (mA)** | **Temps (s)** | **Courant x temps = énergie délivrée** |
|---|---|---|---|
| Stylet | 0,2 à 0,5 mA | 50 à 20 | 10 |
| Visage | 0,5 à 2 mA | 240 à 60 | 120 |
| Sensible | 1 à 4 Ma | 240 à 60 | 240 |
| Jambes | 2,5 à 10 mA | 240 à 60 | 600 |

Le système permet donc de délivrer une quantité d'énergie toujours identique, quelle que soit l'intensité choisie à l'aide de son contrôle de l'énergie délivrée ; cette quantité est définie en fonction du patch connecté à l'aide de son système d'identification et de la régulation du temps d'application.

Le stylet de précision 9 est utilisé pour traiter des zones précises et celui-ci peut être équipé de quatre têtes différentes en fonction de la zone à traiter : la tête large est utilisée pour la moustache et la tête plus fine pour des poils isolés. L'utilisation d'un gel conducteur peut améliorer la conductivité du stylet de précision ou celle des patchs.

### Légende

- 1: Fil raccordé au patch de contact
- 2: Fil raccordé à la résistance du circuit oscillant
- 3: Fil de détection et relié aux patchs pour épilation par électrolyse
- 4: Unité de gestion centrale
- 5, 6, 7: Soquet de transfert (résistances de reconnaissance)
- 8: Patchs pour épilation par électrolyse
- 9: Stylet de précision
- 10: Patchs de contact fermant le circuit

## Revendications

1. Dispositif d'épilation par électrolyse comportant une unité de gestion centrale (4) équipée d'un microprocesseur, et des patchs d'épilation (8) de taille variable adaptés aux différentes parties du corps à traiter ainsi que des patchs de contact (10) permettant de créer un courant d'électrolyse sur la peau, **caractérisé en ce que** ledit dispositif comporte un système d'identification des patchs d'épilation (8) permettant au microprocesseur de l'unité de gestion centrale (4) de les identifier et de réguler la durée d'application du courant en fonction de l'intensité choisie par l'utilisateur afin d'administrer une dose d'énergie spécifiquement associée à la taille du patch d'épilation utilisé(8).

2. Dispositif d'épilation selon la revendication 1, **caractérisé en ce qu'**il comporte en outre un stylet de précision (9).

3. Dispositif d'épilation selon la revendication 2, **caractérisé en ce que** ledit stylet de précision possède plusieurs têtes d'application interchangeables.

4. Dispositif d'épilation selon la revendication 1, **caractérisé en ce que** l'unité centrale (4) possède un système visuel permettant à l'utilisateur d'identifier les parties traitées.

5. Dispositif d'épilation selon la revendication 1, **caractérisé en ce que** ledit dispositif de d'identification (5, 6, 7) comporte un circuit oscillant.

6. Dispositif d'épilation selon la revendication 1, **caractérisé en ce que** ledit dispositif de d'identification (5, 6, 7) comporte des résistances.

7. Procédé de traitement cosmétique à l'aide du dispositif de la revendication 1 comportant les étapes suivantes :
- génération d'un courant continu de faible intensité par une unité de gestion centrale,
- identification du patch d'épilation (8) via le système d'identification du dispositif de la revendication 1,
- adaptation de l'intensité du courant par l'utilisateur en fonction de sa propre sensibilité,
- réglage automatique de la durée de traitement par l'unité de gestion centrale (4).

8. Utilisation du dispositif selon la revendication 1 pour l'épilation durable.
